# EUROPEAN PATENT SPECIFICATION

(11) **EP 2 021 355 B1**
(45) Date of publication and mention of the grant of the patent: **01.09.2010**
(21) Application number: 07748761.9
(22) Date of filing: 11.05.2007
(51) Int. Cl.: C07K 7/62

(54) **METHOD OF POLYMYXIN B RECOVERY FROM FERMENTATION BROTH**
VERFAHREN ZUR RÜCKGEWINNUNG VON POLYMYXIN B AUS EINER FERMENTATIONSBRÜHE
PROCÉDÉ DE RÉCUPÉRATION DE LA POLYMYXINE B À PARTIR D'UN BOUILLON DE FERMENTATION

(30) Priority: 02.06.2006 SK 832006
(43) Date of publication of application: 11.02.2009
(73) Proprietor: Biotika A.S., 976 13 Slovenská Lupca (SK)
(72) Inventor: VARGA, Ivan, 920 01 Hlohovec (SK); BOBÁLOVÁ, Mária, 974 11 Banská Bystrica (SK); MICHALKOVÁ, Eva, 974 04 Banská Bystrica (SK); JAKUBCOVÁ, Mária, 974 11 Banská Bystrica (SK)
(74) Representative: Dolanska, Elena
(86) International application number: PCT/SK2007/050009
(87) International publication number: WO 2007/142611

(56) References cited:
- WO-A1-01/26673
- GB-A- 991 602
- GB-A- 1 089 765
- US-A- 3 132 994
- US-A1- 2006 004 185
- KOBAYASHI M ET AL.: "FR207944, an Antifungal Antibiotic from Chaetomium sp. No. 217- II. Isolation and Structure Elucidation" BIOSCIENCE, BIOTECHNOLOGY, AND BIOCHEMISTRY, vol. 69, no. 5, 2005, pages 1029-1032, XP002447295
- ANONYMOUS: "Sepabeads SP-207" INTERNET ARTICLE, [Online] XP002447299 Retrieved from the Internet: URL:http://www.sigmaaldrich.com/catalog/se arch/ProductDetail/SUPELCO/13623-U> [retrieved on 2007-08-20]
- ROOM AND HAAS COMPANY: "Amberlite XAD 4: Product Data sheet" INTERNET ARTICLE, [Online] October 2003 (2003-10), XP002447300 Retrieved from the Internet: URL:http://www.advancedbiosciences.com/Bio processing_doc/english/xad4.PDF> [retrieved on 2007-08-20]
- MITSUBUSHI CHEMICAL CORPORATION: "DIAION(R) & SEPABEADS(R) Synthetic Adsorbents (SEPABEADS Product List): Types and properties of Synthetic Adsorbents." INTERNET ARTICLE, [Online] 30 September 2000 (2000-09-30), XP002447301 Retrieved from the Internet: URL:http://www.diaion.com/Index_E.htm> [retrieved on 2007-08-20]

## Description

### Technical field

The invention is related to method of polymyxine B recovery from fermentation broth.

### Background art

The polymyxine is a complex of very similar polypeptide antibiotics isolated from different types of *Bacillus polymyxa* strains and relative species. It is a cyclic polypeptide with free aminoacides groups containing characteristic constituents such as α,γ -aminobutanic acid, L-threonine and fatty acides (6-methyloctanoyl acid, 3-hydroxy-6-metyloctanoyl acid, 6-methylheptanoyl acid, heptanoyl acid, octanoyl acid, nonanoyl acid). This complex was divided by semi - preparative PLRP-S with reverse phase on 7 components (Orwa J.A., Govaert C., Busson R., et all, 2001, J.Chromatography A, 912, 369-373). The structure was characterized by methods ¹H- and ¹³C -NMR and molecular weight was measured by mass spectroscopy method. The structure of polymyxine was B₁, Ile-B₁, B₂, B₃ and B₄. Two further components, polymyxine B₅ and B₆, were isolated for the first time and characterized at this study.

The polymyxine B and E have lower toxicity than another polymyxine and they are preferred for medical use, mainly antibacterial more effective polymyxine B.

The procedures for recovery of polymyxine from fermentation broth in principal are based on application of activated carbon and ions exchange. US Patent 2 565 057 described a character of polypeptide antibiotic base as a metabolic product of *Bacillus aerosporus* and *Bacillus polymyxa,* also listed are methods of his recovery from cultivation media by using the absorption character of activated carbon. The first part of impurities are separated from the filtrate of the broth by activated carbon in acid area (pH 2,5). The discolored solution of antibiotic is adjusted to neutral area and than it is adsorbed on active carbon. The adsorbed antibiotic is eluated by an aqueous solution of acid acetone. The eluate is purified by adjustment of pH, separation of acetone and re-adsorption on activated carbon and neutralization of eluate with CaCO₃ and finalization by lyophilisation of the antibiotic solution. Another alternative of antibiotic purification from eluate is a preparation of its insoluble salts such as coagulation by picric acid, and reconverting this salt into aqueous soluble salts usually in a form of sulphates (M. Harold et all.: Antibiotika 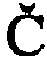 sAV Praha, 1957, page 285-288).

In further 2 patents (GB 742 589 and GB 782 926) the filtrates from the fermentation broth are purified in weak acid ions exchangers in different column systems. The adsorbed polymyxine is eluated by solution of mineral acid or buffer solution with pH in a range from 3 to 5. The obtained eluate is purified on anion exchanger or in strong acid cation exchanger. This repurified eluate is concentrated and polymyxine is coagulated by addition of alcaline solution. Coagulation is running in a range of temperature from 60 °C to 90 °C.

US patent 3 132 994 (1964) describes the method of increasing purification crude sulphates - and N - methylsulphonates of polymyxine B and E. The background of this method is oxidation of this salts solutions in acid or neutral pH (pH 3,5 to 7,5) with 1% water solution of KMnO4. In the next step the low molecular weight fabrics are separated, including Mn - cations in a strong acid cation exchanger in H⁺ cycle, where the polymyxine is not adsorbed.

The procedure for recovery and purification of amphotem and alcaline antibiotics including polymyxine B, using reactive extraction into organic solvent limited with aqueous mixture and extraction in a present of holders di-(2-ethylhexyl)-ester or dinonyl ester of phosphoric acid is described in patent GB 979 887 (1964). Polymyxine B is extracted from the broth into 1% solution of di-(2-ethylhexyl)-ester of phosphoric acid in buthylacetate at pH 7. Purification of polymyxines B and E from acid solutions by alcaline reagent in the presence of chelating agents to form complexes of cations Ca, Mg, Mn, Fe is described in patents GB 1089765 (1967) and US 3413398. The antibiotic is a precipitate in a form of base solution by using alcaline agents such as hydroxides and carbonates of alcaline metals and ammonium hydroxide at pH 8,5 to 11 and addition of chelating agents to avoid coprecipitation of impurities. There is described the whole list of chelating agents, which are used to be added in equivalent ratio to present polyvalent inorganic ions. The precipitation of antibiotic base can be done in 10% water solution of acetone. The examples of these patents describe methods for purification of polymyxine B, wherein the acid solution of crude polymyxine B is oxidized with KMnO₄, according to the patent GB 991602. The leftover of KMnO₄ is eliminated by H₂O₂. The further is continued in precipitation of base in the presence of ethylenediamine - N, N, N, N' tetraacetic acid tetra sodium salt. Base is filtered off, washed and vacuum-dried. The base is converted to the sulphate of polymyxine B by molar equivalent of H₂SO₄.

In described procedures of recovery of polymyxine B solid substance from the broth, the broth is purified by complicated methods and crude antibiotic is obtained. The crude antibiotic must be purified in several steps to get a pure substance.

### Disclosure of the invention

Listed disadvantages are eliminated by the method of polymyxine B recovery from fermentation broth according to the invention as defined in claims 1 to 5, in accordance with claims 1 to 5, the filtrate of the fermentation broth is purificated on non-ionogenic synthetic adsorbents of polystyrene types, polymyxine B is eluated from adsorbent by aqueous solution of organic solvent, the base of polymyxine B is precipitated from eluate, then it is converted by mineral acid to mineral salt of polymyxine B, from which a crystal substance is obtained by drying.

The biomass and insoluble components of cultivation media are separated. The obtained filtrate is adjusted to a pH value from 2 to 6 and eventually discolored by activated carbon in an amount of 1 to 5 g per 1dm³ of filtrate before the next step. Discolored or un-discolored filtrate of a pH 2 to 6, preferable pH 2,5 till 3,5 is purified on non-ionogenic synthetic polystyrene adsorbents with a specific surface of 500 to 1000 m².g⁻¹ and a pore radius of 3 to 30 nm.

The adsorbed polymyxine B is eluated by non-acid or acid aqueous solution of organic solvent at a concentration of 20 to 60% at a pH 2 to 4, particularly with lower alcohol or ketone, preferable with methanol, ethanol, propanol, secondary propanol and acetone.

From the obtained water-organic eluate, or from the eluate after partial or total separation of the organic solvent, polymyxine B base is coagulated by addition of a water solution of mineral alkaline agent, preferable NaOH, KOH, NH₄OH at a pH 8 to 12, preferably at a pH 9 to 11. The base is coagulated at a temperature of 20 to 60°C. The coagulated polymyxine B base is filtered off and washed by deionized water at a temperature of 30 to 80°C. Into washed polymyxine B base, or eventually into water suspension of base, a solution of mineral acid is added, such as HCl, H₂SO₄, to a pH value 5 to 7,5. The obtained solution of polymyxine B is eventually discolored by activated carbon in an amount of 0,1 to 0,5 g per g of polymyxine B and the pure solution of substance is dried.

The invention is supplemented by examples.

### Examples of embodiments

### Example 1

The biomass was separated by centrifugation from 2,1 dm³ of fermentation broth with 3,1 g of polymyxine B. Supernatant was acidified with 10 % solution of H₂SO₄ to pH 3, activated carbon was added in amount 4 g and after 30 minutes suspension was filtered out. Discolored clear filtrate was percolated through column fulfilled with 500 ml of non-ionogenic adsorbent Diaion HP 21, with flow rate 2 dm³.h⁻¹. The column was washed after adsorption by 500 ml of deionized water. The adsorbed polymyxine B was eluated with 40 % aqueous solution of acetone under flow rate 0,5 dm³ .h⁻¹. It was obtained 0,84 dm³ of eluate with amount 2,2 g of polymyxine B. Into the eluate was added under very slow agitation at laboratory temperature 20 % aqueous solution of NaOH to a value pH 11. After 2 h of crystallization the polymyxine B base was filtered off and washed with 2 dm³ of deionized hot water at the temperature 70 °C. It was obtained 20 g of moist product of polymyxine B base, which was mixed together with 5 ml of deionized water and 10 % solution of H₂SO₄ was slowly added till polymyxine B sulphate occurred. Into 50 ml of polymyxine sulphate solution at a pH 6 was added 0,5 g of activated carbon and after 45 min suspension was filtered off. Discolored solution was than filtered through the membrane with fiber size 0,45 µm and 0,22 µm. The product was recovered by freeze-drying. It was obtained 1,7 g of polymyxine B sulphate with purity 7900I.U./ mg

### Example 2

From the eluate which was prepared according to example 1, acetone was separated by vacuum distillation. It was obtained 0,5 dm³ of concentrate, which was adjusted to pH 7,5 with concentrated NH₄OH at the temperature 60°C and then 20 % solution of NaOH was added to reach pH 12. After 2 hours of crystallization at a temperature 40°C the polymyxine base was obtained, which was filtered off and washed 5 times with 0,3 dm³ of deionized water at the temperature 30 °C. It was obtained 15 g of moistly coagulate, which was recovered according to example 1. It was obtained 1,3 g of polymyxine B sulphate with purity 8100 I.U./mg.

### Example 3

Procedure according to example 1 with following difference that it was used 60 % ethanol for elution. It was obtained 0,58 dm³ of eluate, which was recovered according to example 1 with the difference, that polymyxine base was precipitated by NH₄ OH to pH 8. It was obtained 1,2 g of polymyxine B sulphate with purity 7200 I.U./mg

### Example 4

From 4 dm³ of fermentation broth with an amount of 4,4 g of polymyxine B and the pH 6 was obtained by micro filtration 4,2 dm³ of clear permeate, which was acidified by H₂SO₄ at the pH 3. Into the solution 21 g of activated carbon was added and after 1 hour of mixing the suspension was filtered off. Discolored permeate was percolated through the column fulfilled by 1,2 dm³ of non-ionogenic adsorbent Amberlite XAD-4. After washing of column by deionized water the polymyxine B was eluated by 60 % water solution of secondary propanol. It was obtained 1, 2 dm³ of eluate, which was adjusted to pH 10.8 by solution of NaOH. The base which was obtained was separated by centrifugation and washed by hot deionized water while the pH of washed water is 7,8. It was obtained 25 g of moisty base which was mixed together with 10 ml of deionized water. The solution of 10 % H₂SO₄ was slowly added till polymyxine B sulphate solution was obtained at the pH 7. Solution of polymyxine B sulphate (70 ml) was discolored by 0,3 g of active carbon, filtered through the membrane 0,22 µm and lyofilizated. It was obtained 2,4 g of polymyxine B sulphate with purity 7700 I.U./ mg.

### Industrial applicability

The method of polymyxine B recovery according to invention is useful for the recovery of a pure antibiotic from fermentation broth.

## Claims

1. The method for recovering polymyxine B from fermentation broth **characterized in that** the filtrate, which is obtained from fermentation broth, is purified on non-ionogenic synthetic adsorbents of the polystyrene type with a specific surface of 500 to 1000 m².g⁻¹ and a pore radius of 3 to 30 nm, polymyxine B is eluated from said adsorbent by an aqueous solution of organic solvent, the polymyxine B is coagulated from the eluate and converted by mineral acid into polymyxine B solution, from which polymyxine B is dried until it is crystallized.

2. The method according to claim 1 **characterized in that** the filtrate, which is obtained from fermentation broth, is adjusted to a pH value of 2 to 6 and, eventually, is discolored by activated carbon in an amount of 1 to 5 g on 1 dm³ of filtrate.

3. The method according to claim 1 and 2 **characterized in that** the water solution of organic solvent which is used for eluation of polymyxine B from adsorbent is lower alcohol or ketone such as methanol, ethanol, propanol, secondary propanol, acetone with a concentration 20 of 60 % by weight.

4. The method according to claim 1 to 3 **characterized in that** the polymyxine B base is coagulated from aqueous - organic eluate or from eluate after partial or total separation of organic solvent by pH adjustment of solution to 8 to 12 at a temperature of 20 to 60°C.

5. The method according to claim 1 to 4 **characterized in, that** the polymyxine B base is converted into polymyxine B by addition of mineral acid until the pH of solution is 5 to 7,5.

## Patentansprüche

1. Methode zur Aufbereitung von Polymyxin B aus der Gährungsflüssigkeit **dadurch gekennzeichnet, dass** das Filtrat das aus der Gährungsflüssigkeit gewonnen wird, auf nichtionogenen synthetischen Adsorbern des Polystyroltyps mit einer spezifischen Oberfläche von 500 to 1000 m².g⁻¹ und einem Porenradius von 3 bis 30 nm purifiziert ist, wobei das Polymyxin B von den oben genannten Adsorbern durch eine wässrige Lösung ein organisches Lösungsmittel eluiert wird, das Polymyxin B wird aus dem Eluat zur Gerinnung gebracht und dann mittels Mineralsäure in eine Polymyxin B Lösung umgewandelt, aus der dann das Polymyxin B so lange getrocknet wird, bis es kristallisiert.

2. Die Methode nach dem Anspruch 1 **dadurch gekennzeichnet, dass** das Filtrat, das aus der Gährungsflüssigkeit gewonnen wird, auf einen pH-Wert von 2 bis 6 eingestellt wird und schließlich durch Aktivkohle in einer Menge von 1 bis 5 g per 1 dm³ des Filtrats entfärbt wird.

3. Die Methode nach den Ansprüchen 1 und 2 die sich **dadurch gekennzeichnet, dass** die wässrige Lösung des organischen Lösungsmittels, die zur Eluierung des Polymyxin B von den Adsorbern genutzt wird, aus einem niedrigeren Alkohol oder aus Ketonen besteht wie z. B. Methanol, Ethanol, Propanol, sekundäres Propanol, Azeton mit einer Konzentration von 20 bis 60 Masseprozent.

4. Die Methode nach den Ansprüchen 1 bis 3 die sich **dadurch gekennzeichnet, dass** die Polymyxin-B-Base aus dem wässrigen organischen Eluat oder aus dem Eluat koaguliert wird nach einer teilweisen oder vollständigen Abscheidung des organischen Lösungsmittels durch eine pH-Wert Anpassung der Lösung auf 8 bis 12 bei einer Temperatur von 20 bis 60 °C.

5. Die Methode nach den Ansprüchen 1 bis 4 die sich **dadurch gekennzeichnet, dass** die Polymyxin-B-Base durch den Zusatz von organischer Säure in Polymyxin B umgewandelt wird, bis der pH-Wert der Lösung den Wert 5 bis 7,5 erreicht.

## Revendications

1. L'isolation de la polymyxine B du sol ferménté **caractérisée par le fait que** le filtrat gagné du sol fermenté est purifié sur les adsorbants non ionogènes synthétiques du type polystyrène avec une surface spécifique de 500 à 1000 m².g⁻¹ et la taille des pores entre 3 et 20 nm, la polymixine B étant éluée par la solution aqueuse d'un dissolvant organique ; c'est la base de polymyxine B qui est séparée par la précipitation : cette base est convertie par un acide minérale en solution de polyxymine B qui sera séchée ce qui prouira la substance crystalline.

2. Revendication de brevet conforme au point 1 **caractérisée par le fait que** le filtrat gagné du sol fermenté est traité pour obtenir une valeur pH de 2 à 6 et éventuellement décoloré par le charbon actif dont la quantité fait 1 à 5 g par 1 dm³ du filtrat.

3. Revendication de brevet conforme aux points 1 et 2 **caractérisée par le fait que** la solution aqueuse du dissolvant organique, utilisée pour l'élution de la polyxymine B de l'absorbant est un alcool moins complexe ou la cétone, par ex. méthanol, éthanol, alcool propylique secondaire, acétone dont la concentration sera entre 20 et 60 % de poids.

4. Revendication de brevet conforme aux points 1 et 3 **caractérisée par le fait que** la base de polyxymine B sera précitipitée de l'éluat aqueux organique ou bien d'un éluat après l'achèvement de la séparation partielle ou complète du dissolvant organique par la modification de pH de la solution à une valeur entre 8 et 12 à la température de 20 à 60 °C.

5. Revendication de brevet conforme aux points 1 à 4 **caractérisée par le fait que** la base de polyxymine B sera convertie en polyxymine B par un acide minéral ajouté jusqu'à une valeur pH de la solution entre 5 et 7,5.
